# EUROPÄISCHE PATENTSCHRIFT

(11) **EP 4 009 918 B1**
(45) Veröffentlichungstag und Bekanntmachung des Hinweises auf die Patenterteilung: **18.12.2024**
(21) Anmeldenummer: 20746637.6
(22) Anmeldetag: 27.07.2020
(51) Int. Cl.: A61F 2/82, A61F 2/86, A61F 2/90, A61F 2/91, A61F 2/915, A61F 2/962, A61M 25/00, A61M 25/01

(54) **IMPLANTAT MIT EINER DREIDIMENSIONALEN STRUKTUR**
IMPLANT HAVING A THREE-DIMENSIONAL STRUCTURE
IMPLANT AVEC UNE STRUCTURE TRIDIMENSIONELLE

(30) Priorität: 05.08.2019 EP 19190021
(43) Veröffentlichungstag der Anmeldung: 15.06.2022
(73) Patentinhaber: Biotronik AG, 8180 Bülach (CH)
(72) Erfinder: KOOP, Karsten, 18057 Rostock (DE); ROTHSTOCK, Stephan, 13353 Berlin (DE); HEIN, André, 18055 Rostock (DE); GOEBEL, Paul, 18059 Rostock (DE); FLORES, Imanol, 18059 Rostock (DE); HOF, Andreas, 23568 Luebeck (DE)
(74) Vertreter: Biotronik Corporate Services SE
(86) Internationale Anmeldenummer: PCT/EP2020/071119
(87) Internationale Veröffentlichungsnummer: WO 2021/023545

(56) Entgegenhaltungen:
- EP-A1- 2 591 751

## Beschreibung

Die vorliegende Anmeldung betrifft eine röhrenförmige durchbrochene Struktur für eine Katheterhülse oder für ein Implantat, die sich in einer Längsrichtung oder Umfangsrichtung erstreckt und aus einer Vielzahl von Stegen zusammengesetzt ist, die zumindest teilweise in Längsrichtung oder Umfangsrichtung verlaufen. Die Erfindung betrifft ferner eine entsprechende Katheterhülse, ein entsprechendes Implantat sowie einen entsprechenden Katheter.

Medizinische Implantate, insbesondere intraluminale Endoprothesen, für die unterschiedlichsten Anwendungen sind in großer Vielfalt aus dem Stand der Technik bekannt. Als Implantat im Sinne der vorliegenden Erfindung sind endovaskuläre Prothesen oder sonstige Endoprothesen, beispielsweise Stents (Gefäß-Stents (vaskuläre Stents, einschließlich Stents für die Anwendung im Bereich des Herzens und Herzklappen-Stents, zum Beispiel Mitralklappen-Stents, Pulmonalklappen-Stents) und Gallengang-Stents), Endoprothesen zum Verschließen von persistierenden Foramen Ovale (PFO), Stentgrafts zur Behandlung von Aneurysmen, Endoprothesen zum Verschließen eines ASG (Vorhofscheidewanddefekt, Atrioseptaldefekt) sowie Prothesen im Bereich des Hart- und Weichgewebes zu verstehen.

Ein derartiges Implantat nimmt üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels eines Katheters in das zu behandelnde Gefäß oder Organ durch enge Gefäße hindurch eingeführt und an der zu behandelnden Stelle positioniert werden. In dem expandierten Zustand verbleibt das Implantat in dem Gefäß oder Organ und ist dort festgelegt, nachdem der Katheter aus dem Körper des Behandelten entfernt wurde. Beispielsweise wird bei einer Transkatheter-Aortenklappenimplantation (endovaskulärer Aortenklappenersatz, englisch transcatheter aortic valve implantation, TAVI) eine künstliche Aortenklappe in einem röhrenförmigen Gerüst in das Herz eingebracht. Mittels Katheter wird die Klappe in Position gebracht. Anschließend wird sie entfaltet und verankert. Die körpereigene Aortenklappe wird dabei nicht entfernt, sondern durch das Implantat verdrängt. Bei einem selbstexpandierenden Implantat aus einer Formgedächtnislegierung geht das Implantat bei Überschreiten einer Umwandlungstemperatur oder bei einer bestimmten Spannung selbstständig in den expandierten Zustand über. Bei einem Implantat mit einem ballonexpandierbaren Grundgerüst (Stent) ist hierfür ein Ballon nötig.

Aus der Druckschrift US 2008/0188928 A1 ist ein Katheter zum Freisetzen eines Herzklappenimplantats bekannt, das für den Transport des zusammengelegten Herzklappenimplantats durch die Gefäße des Patienten eine Kapselhülse aufweist, die einerseits flexibel ist, um durch die gewundenen Gefäße hindurch geführt werden zu können, und andererseits dafür geeignet ist, das Implantat aufzunehmen und zu halten sowie die Freisetzung des Implantats an der Behandlungsstelle zu ermöglichen. Die Hülse setzt sich zusammen aus einer inneren Polymerschicht und einer äußeren Polymerschicht, zwischen denen ein Stützelement oder mehrere Stützelemente angeordnet sind, welche eine variable axiale Steifigkeit besitzen. Ein röhrenförmiges Stützelement ist beispielsweise aus einer Vielzahl von Ringen oder Rippen gebildet, welche in Längsrichtung nebeneinander angeordnet sind. Alle Rippen sind durch einen in Längsrichtung verlaufenden Steg verbunden.

Die Druckschriften EP 2 591 751 A1, EP 2 679 198 A1 und US 2010/0249905 A1 zeigen Implantate mit verschiedenen durchbrochenen röhrenförmigen Strukturen. Die Druckschrift EP 2 679 198 A1 beschreibt hierbei einen Stent für ein Herzklappenimplantat bestehend aus einer Drahtstruktur mit mehreren, in Längsrichtung nebeneinander angeordneten Abschnitten die sich jeweils im Aufbau und in ihren Eigenschaften voneinander unterscheiden und miteinander verbunden sind. Die Druckschrift US 2010/0249905 A1 beinhaltet demgegenüber ein Implantat, das röhrenförmig gestaltet ist und eine Vielzahl von Stegen aufweist, die mittels schräg verlaufenden flexiblen Konnektoren verbunden sind. Die Stege und Konnektoren besitzen Öffnungen, die mit einem Medikament befüllt sind für die Freisetzung an der Stelle des Körpers, in die das Implantat eingesetzt ist.

EP 2 591 751 A1 beschreibt ein endoluminales Prothesensystem für ein verzweigtes Körperlumen umfassend eine Gefäßprothese (11). Die Gefäßprothese (11) ist innerhalb einem verzweigten Gefäßlumen entfaltbar und umfasst einen Stent (48) mit einem im Allgemeinen röhrenförmigen Körperabschnitt (33), einem aufweitbaren proximalen Endabschnitt (36) und einem Kopplungsabschnitt (38), der zwischen dem Körperabschnitt und dem aufweitbaren Abschnitt angeordnet ist. Der Kopplungsabschnitt ist vorzugsweise quetschfester als der Körperabschnitt.

Für das Implantieren von stentgestützten Herzklappenimplantaten werden heutzutage vorwiegend Katheter aus Kunststoffen beziehungsweise Komposits eingesetzt, deren Biegsamkeit und Flexibilität begrenzt sind. Während der Implantation und Positionierung des Herzklappenimplantats wird dieses aus einer Katheterhülse (auch als Kapsel bezeichnet) freigesetzt, welche das Implantat während des Transports durch die Gefäße des Patienten im komprimierten Zustand gehalten hat. Derartige Herzklappenimplantate bestehen aus einem Gerüst, welches wie ein Stent ausgestaltet ist, welches das eigentliche Klappenmaterial trägt. Dieses Gerüst bzw. der Stent wird selbstexpandierend, z.B. aus einem Formgedächtnismaterial wie Nitinol, ausgeführt und von der Katheterhülse in seinem komprimierten Zustand gehalten. Durch eine Relativbewegung der Katheterhülse zu dem selbstexpandierenden Herzklappenimplantat fällt die komprimierende Kraft weg und der selbstexpandierende Stent bzw. das Gerüst, und damit das gesamte Herzklappenimplantat, wechselt vom komprimierten Zustand auf den expandierten Zustand. Das Implantat muss jedoch auch teilweise wieder zurück in die Katheterhülse gezogen werden, um eine Repositionierung des Implantats zu ermöglichen.

Insbesondere bei selbstexpandierenden Herzklappenimplantaten treten beim Freisetzen und beim Zurückziehen des Implantats in die Katheterhülse starke radiale und axiale Kräfte auf, welche abhängig von der Steifigkeit des Implantats sind. Diese Reaktionskräfte können zu bleibenden Verformungen der Katheterhülse führen, welche beim Entfernen des Katheters mit der Katheterhülse aus dem Körper des Behandelten zu Verletzungen in den Gefäßen und entsprechenden Komplikationen führen können. Auch bei Implantaten ist eine höhere Biegeflexibilität erwünscht.

Die Aufgabe der vorliegenden Erfindung besteht somit darin, eine Struktur anzugeben, welche in radialer Richtung flexibel ist und gleichzeitig die Übertragung von starken radialen und axialen, d.h. in Längsrichtung verlaufenden, Druckkräften ermöglicht.

Die obige Aufgabe wird gelöst durch die Struktur mit den Merkmalen des Anspruchs 1. Beziehungsweise wird die Aufgabe gelöst durch das Implantat umfassend die besagte Struktur gemäß Anspruch 1.

Die röhrenförmige durchbrochene Struktur für eine Katheterhülse oder für ein Implantat mit einer Vielzahl von Stegen, die zumindest teilweise in Längsrichtung oder Umfangsrichtung verlaufen, weist erfindungsgemäß mindestens ein Gelenkelement auf, dessen in Längsrichtung verlaufender Hauptsteg einen durchgehenden Spalt (Trennung) besitzt. Weiterhin ist (sind) an dem Gelenkelement zudem mindestens einen Brückensteg, vorzugsweise zwei Brückenstege, vorgesehen, wobei jeder Brückensteg in Umfangsrichtung neben dem Hauptsteg angeordnet sowie in Längsrichtung vor und hinter dem Spalt mit dem Hauptsteg verbunden ist.

Im Bereich des Spalts ist der Hauptsteg vollständig durchtrennt. Vorzugsweise ist der Spalt etwa in der Mitte des Gelenkelements angeordnet. Der oder die Brückensteg(e) wirken als Verbinder und verhindern ein Auseinanderdriften des Hauptstegs bei leichten Zugkräften. Das Gelenkelement ist mit den Stegen der Struktur verbunden, vorzugsweise - in Längsrichtung gesehen - an beiden Enden jeweils mit einem in Längsrichtung verlaufenden Steg der Struktur, besonders bevorzugt in dem Bereich, in dem der oder die Brückensteg(e) mit dem Hauptsteg verbunden sind. Dieser Verbindungssteg bildet in einem besonders bevorzugten Ausführungsbeispiel die Fortsetzung des Hauptstegs in Längsrichtung.

Im Rahmen der vorliegenden Erfindung werden unter in Längsrichtung verlaufenden Stegen Stege verstanden, welche sowohl exakt parallel als auch unter einem kleinen Winkel zur Längsrichtung der Struktur verlaufen. Beim Abrollen der auf dem Umfang der Röhre liegenden Struktur in eine Ebene beträgt der Winkel zwischen dem Steg und der Längsrichtung maximal 0-45°. Die Längsrichtung einer Struktur wie bei einem im Wesentlichen zylindrischen Implantat bzw. einer Katheterhülse entspricht der Zylinderachse der zylindrischen Implantatstruktur bzw. der Katheterachse bei einer Katheterhülse.

Das erfindungsgemäße Gelenkelement ist eine Struktur, welche in ihren Eigenschaften mit einem Festkörpergelenk verglichen werden und ähnlich wie menschliche Gelenke Druckkräfte übertragen kann, jedoch auch ein Gleiten im Bereich des im Hauptsteg angeordneten Spalts sowie eine Verdrehung der im Spalt gegenüber liegenden Enden des Hauptstegs zueinander erlaubt. Daher weist das Gelenkelement eine hohe Biegeflexibilität auf. Das Gelenkelement ist ferner in der Lage, Druckkräfte, die in Längsrichtung oder in radialer Richtung verlaufen, zu übertragen. Dies erfolgt einerseits über die Brückenstege. Andererseits werden bei Überschreiten eines vorgegebenen axialen Drucks die den Spalt ausbildenden Enden des Hauptstegs soweit zusammengedrückt, dass diese aneinander anliegen. Die Druckübertragung erfolgt dann nicht nur über den oder die Brückensteg(e), sondern auch über den Hauptsteg. Durch das erfindungsgemäße Gelenkelement, welches vorzugsweise in einer Vielzahl in der Struktur vorliegt, erhält die Struktur die gewünschte Biegeflexibilität, wobei auch große radiale und axiale Druckkräfte übertragen werden können. Bei Stentimplantaten kann die Gelenkstruktur auch an den Stegkreuzungen in Umfangsrichtung eingesetzt werden. Dadurch bekommt der Stent eine große Biegeflexibilität, welche das Anpassen an anatomische Strukturen wie Verkalkungen erleichtert. Die Radialkraft kann so über die sich schließenden Gelenke übertragen werden, während der Stent gleichzeitig in Umfangsrichtung eine hohe Flexibilität (geringe Crush Resistance) besitzt, welche notwendig ist, um besonders anpassungsfähig zu sein.

In einem bevorzugten Ausführungsbeispiel ist der Brückensteg halbkreisförmig, U-förmig, V-förmig oder mäanderförmig ausgebildet. Die unterschiedlichen Formen der Brückenstege erlauben eine Anpassung an die von der Struktur geforderte unterschiedliche Biegeflexibilität abhängig von der Verwendung der Struktur. In einer weiteren Ausführungsform können die Brückenstege zusätzliche Federelemente oder Veränderungen des Querschnitts aufweisen.Eine höhere Biegeflexibilität kann dadurch erreicht werden, dass der Brückensteg mindestens einen Einkerbungsabschnitt mit einer quer zur Längsrichtung verlaufende Einkerbung aufweist. Ein derartiger Einkerbungsabschnitt kann als U-, V- oder W-förmiger Abschnitt ausgebildet sein. Alternativ kann der Brückensteg in dem Abschnitt lediglich einen Bereich mit einer reduzierten Breite oder Dicke aufweisen.

Es ist von Vorteil, wenn der Spalt eine Breite von mindestens 20 µm, vorzugsweise von mindestens 40 µm bis 500 µm je nach Anwendungsfall aufweist. Hierbei wird die Spaltbereite in Richtung der Längsachse (d. h. in Längsrichtung) der röhrenförmigen Struktur gemessen. Hierdurch wird die gewünschte erhöhte Biegeflexibilität gewährleistet.

Ebenfalls vorteilhaft für die Biegeflexibilität des Gelenkelements ist es, wenn der Brückensteg eine Breite von 20% bis 40% der Haupt-Stegbreite aufweist. Bei Ausgestaltungen mit mehr als einem Brückensteg, wird die Breite der Brückenstege derart gewählt, dass sie in Summe die Breite des Hauptsteges nicht überschreitet. Entsprechend weisen in einer Ausgestaltung mit zwei Brückenstegen diese vorzugsweise jeweils eine Breite von 20% bis 50% der Haupt-Stegbreite auf. Bei einer Ausgestaltung mit nur einem Brückensteg weist dieser eine Breite von 20% bis 100% des Hauptsteges auf. Die Breite wird jeweils senkrecht zur Mittellinie des jeweiligen Stegs gemessen.

Eine Biegeflexibilität in beliebige radiale Richtung wird dann gewährleistet, wenn die Struktur eine Vielzahl von Gelenkelementen aufweist, die in Umfangsrichtung nebeneinander angeordnet sind. Besonders bevorzugt ist die Vielzahl von Gelenkelementen entlang des gesamten Umfangs der Struktur vorgesehen, so dass ein trichterförmiges radiales Aufweiten der Struktur, beispielsweise beim Freisetzen eines selbstexpandierenden Implantats realisiert wird. In Bezug auf eine Katheterhülse ist es insbesondere von Vorteil, wenn ein Abschnitt mit einer Vielzahl von in Umfangsrichtung nebeneinander angeordneten Gelenkelementen einen distalen Abschnitt der Katheterhülse ausbildet, denn insbesondere in dem distalen Abschnitt der Katheterhülse wird für das Freisetzen und Zurückziehen beispielsweise eines Herzklappenimplantats aus/in den Katheter in diesem Bereich eine erhöhte Biegeflexibilität benötigt. Besonders bevorzugt sind in Längsrichtung mindestens zwei solcher Abschnitte mit der Vielzahl von Gelenkelementen in Längsrichtung hintereinander vorgesehen. Hierbei können die Gelenkelemente so ausgebildet sein, dass ihre Flexibilität in proximaler Richtung abnimmt, beispielsweise durch Verstärkung der Brückenstege. In einem bevorzugten Ausführungsbeispiel der erfindungsgemäßen Struktur ist in Längsrichtung benachbart zu dem Abschnitt mit der Vielzahl von nebeneinander angeordneten Gelenkelementen ein Abschnitt vorgesehen, in dem ein oder mehrere wellenförmige Stege, die besonders bevorzugt um den gesamten Umfang umlaufen, angeordnet sind. Diese können die von den Gelenkelementen weitergeleiteten Kräfte aufnehmen. Hierdurch wird eine beim Herausführen des Katheters aus dem Körper problematische Verformung der Katheterhülse vermieden.

In einem Ausführungsbeispiel der vorliegenden Erfindung ist es von Vorteil, wenn die erfindungsgemäße Struktur aus einer Formgedächtnislegierung, insbesondere Nitinol, besteht oder diese umfasst. Eine erfindungsgemäße Struktur aus Polymer, einer Cobalt-Chrom-Legierung (CoCr) oder Stahl kann ebenfalls in bestimmten Ausgestaltungen zweckmäßig sein.

Die obige Aufgabe wird auch durch ein Implantat, insbesondere ein Stent, gelöst, das zumindest in einem Abschnitt die oben beschriebene erfindungsmäße röhrenförmige, durchbrochene Struktur aufweist. In diesem Abschnitt besitzt das Implantat die beschriebene Biegeflexibilität, wobei auch in radialer und axialer Richtung hohe Druckkräfte übertragen werden können. Erfindungsgemäß kann das Implantat auch vollständig aus der beschriebenen, röhrenförmigen, durchbrochenen Struktur mit einem Gelenkelement oder einer Vielzahl von Gelenkelementen, die sowohl in Umfangsrichtung als auch in Längsrichtung hintereinander beziehungsweise nebeneinander angeordnet sind, bestehen. Besonders bei Herzklappenstents kann eine hohe Anpassungsfähigkeit in Umfangsrichtung von Vorteil sein, um sich anatomischen Strukturen wie Verkalkungen anzupassen.

Die obige Aufgabe wird ferner durch eine Katheterhülse gelöst, welche sich insbesondere für das Einbringen eines stentgestützten Herzklappenimplantats eignet. Die erfindungsgemäße Katheterhülse weist eine Versteifungshülse und eine erste Polymerlage auf, die in radialer Richtung innerhalb der Versteifungshülse angeordnet ist. Ferner ist eine zweite Polymerlage vorgesehen, die in radialer Richtung außerhalb der Versteifungshülse angeordnet ist, wobei die obige erfindungsgemäße Struktur einen distalen Abschnitt der Versteifungshülse, der auch als Krone bezeichnet wird, ausbildet. Insbesondere im Bereich der Krone, d.h. im Bereich der erfindungsgemäßen Struktur besteht das Versteifungsröhrchen aus einer Formgedächtnislegierung, vorzugsweise aus Nitinol. Unter einer Versteifungshülse wird entsprechend eine mechanisch stabile Struktur verstanden, die als Teil der Katheterhülse das Implantat wie beispielsweise das stentgestützte Herzklappenimplantat im Einführzustand des Katheters überdeckt und im Falle eines selbstexpandierenden Implantats, dieses in seiner komprimierten Form hält.

Die erfindungsgemäße Katheterhülse, auch als Implantatkapsel bezeichnet, ermöglicht aufgrund ihrer Biegeflexibilität am distalen Ende ein Aufweiten der Katheterhülse zum Freisetzen des darin angeordneten Implantats. Zudem ist auch die Übertragung von axialen Druckkräften beim Resheathen möglich, sodass die Katheterhülse nach dem Resheathen vollständig in seiner Ausgangsform zurückgeht. Insbesondere lässt sich die Struktur wie eine Trompete oder ein Trichter aufweiten, wenn die Krone entlang ihres gesamten Umfangs eine Vielzahl der oben beschriebenen Gelenkelemente, die nebeneinander angeordnet sind, besitzt. Die Brückenelemente bewirken eine Führung des Gelenkelements und bestimmen deren Biegeweichheit. Sie sind jedoch auch in der Lage, leichte Zugkräfte zu übertragen, um ein Auseinanderreißen des Gelenkelements zu verhindern.

Die erfindungsgemäße Katheterhülse weist in einem bevorzugten Ausführungsbeispiel in Längsrichtung mindestens zwei hintereinander angeordnete Abschnitte auf, wobei in jedem Abschnitt eine Vielzahl von Gelenkelementen in Umfangsrichtung nebeneinander angeordnet sind, verteilt über den gesamten Umfang. Die erfindungsgemäße Katheterhülse erlaubt ein problemloses Freilassen und Zurückziehen eines Implantats und kehrt danach problemlos in seine Ausgangsform zurück, sodass Verformungen und somit beim Herausführen der Katheterhülse unerwünschte Wechselwirkungen mit dem Gefäß vermieden werden.

Die obige Aufgabe wird analog durch einen Katheter mit der oben beschriebenen Katheterhülse gelöst, wobei die Katheterhülse zur Aufnahme eines zusammengefalteten Implantats, insbesondere eines stentgestützten Herzklappenimplantats, dient und ausgebildet ist sowie mit dem Außenschaft des Katheters verbunden ist. Das Implantat ist vorzugsweise über einen sogenannten Prothesenkonnektor auf dem Innenschaft des Katheters fixiert. Wie bei herkömmlichen Kathetern ist der Außenschaft auf dem Innenschaft geführt und beweglich.

Weitere Ziele, Merkmale, Vorteile und Anwendungsmöglichkeiten der Erfindung ergeben sich auch aus der nachfolgenden Beschreibung von Ausführungsbeispielen der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch
- Fig. 1: einen erfindungsgemäßen Katheter vor der Implantation eines Implantats in einer perspektivischen Ansicht von der Seite;
- Fig. 2: einen distalen Abschnitt des Katheters aus Fig. 1 in einer perspektivischen Ansicht von der Seite nach dem Freisetzen des Implantats;
- Fig. 3: eine erfindungsgemäße Katheterhülse mit Außenschaft in einer perspektivischen Ansicht von der Seite;
- Fig. 4: einen Querschnitt durch die Katheterhülse gemäß Fig. 3 an der Stelle C (siehe Fig. 3);
- Fig. 5 - 6: jeweils einen distalen Abschnitt der Katheterhülse gemäß Fig. 3 in einer Ansicht von der Seite;
- Fig. 7: den distalen Abschnitt einer Versteifungshülse eines weiteren Ausführungsbeispiels einer erfindungsgemäßen Katheterhülse in einer Ansicht von der Seite;
- Fig. 8: einen Ausschnitt der erfindungsgemäßen Struktur der Versteifungshülse der Katheterhülse gemäß Fig. 3 in einer Ansicht von der Seite;
- Fig. 9: einen Ausschnitt der Struktur am distalen Ende der Versteifungshülse des in Fig. 7 gezeigten Ausführungsbeispiels einer erfindungsgemäßen Katheterhülse in einer Ansicht von der Seite;
- Fig. 10: das Gelenkelement der in den Fig. 8 und 9 im Ausschnitt dargestellten erfindungsgemäßen Strukturen in einer Ansicht von der Seite;
- Fig. 11 - 15: weitere Ausführungsbeispiele für Gelenkelemente erfindungsgemäßer Strukturen in einer Ansicht von der Seite;
- Fig. 16 - 17: ein Implantat, beispielsweise in Form eines Stents oder eines Stentabschnittes, in dem ein Gelenkelement in die röhrenförmige Struktur integriert ist.

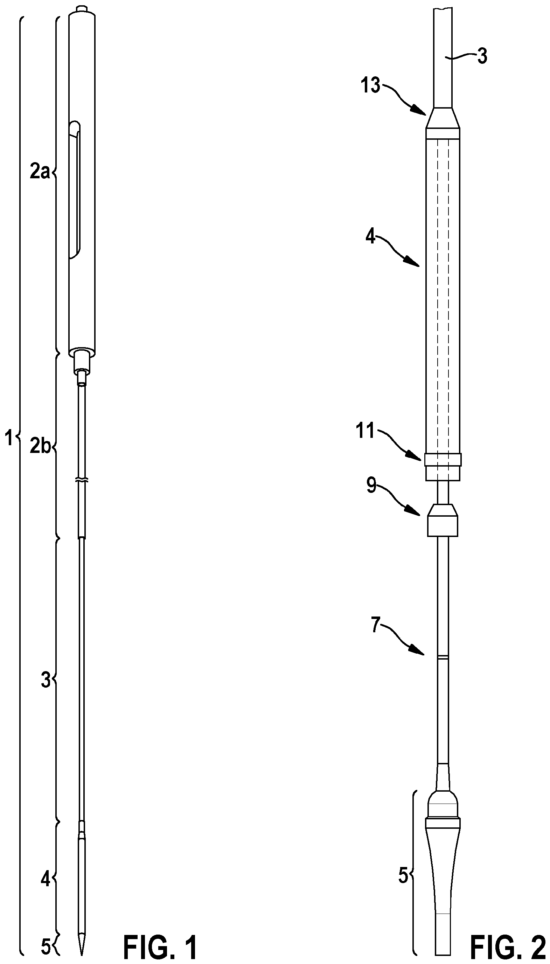

Fig. 1 zeigt einen erfindungsgemäßen Katheter 1 mit einem am proximalen Ende des Katheters angeordneten Handgriff 2a, einen Stabilisierabschnitt 2b, einen Außenschaft 3 sowie einer auf dem Außenschaft 3 angeordneten Katheterhülse 4, wie er beispielsweise zur Implantation eines selbstexpandierenden stentbasierten Herzklappenimplantats verwendet wird. Am äußersten distalen Ende ist eine stumpfe Katheterspitze 5 vorgesehen. Der Stabilisierabschnitt 2 schirmt den zurückziehbaren Außenschaft 3 gegen den Einführschaft (Introducer) und die Gefäßwand ab, sodass der Außenschaft 3 frei zurückgezogen werden kann. Der Handgriff 2a dient dem Beladen, Freisetzen und Zurückziehen eines in der Katheterhülse 4 angeordneten Implantats, beispielsweise eines stentbasierten Herzklappenimplantats. Die Katheterspitze 5 bildet das distale Ende eines innerhalb des Außenschafts 3 angeordneten Innenschafts 7 (siehe Fig. 2), wobei die Katheterspitze 5 vorzugsweise aus PEBAX gefertigt und unter Röntgenbestrahlung sichtbar ist.

Fig. 2 stellt das distale Ende des in Fig. 1 skizzierten Systems nach dem Freisetzen des Implantats dar. In dieser Fig. ist auch zu sehen, dass auf dem Innenschaft 7 ein Prothesenkonnektor 9 angeordnet ist, mit dem das Implantat axial auf dem Innenschaft 7 fixiert ist. Die Katheterhülse 4 weist vorzugsweise an ihrem distalen Ende zur Erleichterung der Beobachtung einen unter Röntgenbestrahlung sichtbaren Ring 11 auf. Die Katheterhülse 4 ist mittels eines proximalen Konnektors 13 mit dem Außenschaft 3 verbunden. Katheterhülse 4 und Außenschaft 3 können jedoch auch in anderen Ausgestaltungen aus einem Stück ausgeführt werden.

Wie bereits oben erläutert wurde, ist das Implantat zunächst in dem in Fig. 1 gezeigten Zustand in der Katheterhülse 4 (auch als Kapsel bezeichnet) im komprimierten Zustand angeordnet und wird durch die Katheterhülse 4 in diesem Zustand gehalten. Die Katheterhülse 4 ist über den Außenschaft 3 mit dem Handgriff 2a verbunden. In diesem Zustand erfolgt der Transport des in der Katheterhülse 4 fixierten und komprimierten Implantats durch die Gefäße des Patienten an den Ort der Behandlung.

Zum Freisetzen des Implantats wird die Katheterhülse 4 nach proximal zurückgezogen. Der Rückzug wird über den Handgriff 2a ausgelöst und über den Außenschaft 3 auf die Katheterhülse 4 übertragen. Hierbei wird zunächst lediglich ein kurzer distaler Abschnitt des Implantats freigesetzt und der Sitz geprüft. Bei ungünstiger Positionierung wird die Katheterhülse mittels des Handgriffs 2a wieder nach distal zurückgeschoben, sodass das Implantat wieder durch die Katheterhülse 4 bedeckt und vollständig in den komprimierten Zustand übergegangen ist. Nun wird der Katheter 1 repositioniert und das Freisetzen des in der Katheterhülse 4 angeordneten Implantats beginnt von neuem. Um Verformungen der Katheterhülse 4 während des Freisetzens und gegebenenfalls Zurückziehens der Katheterhülse 4 zu vermeiden, muss diese besonders biegeelastisch sein und zudem axiale und radiale Druckkräfte gut übertragen können.

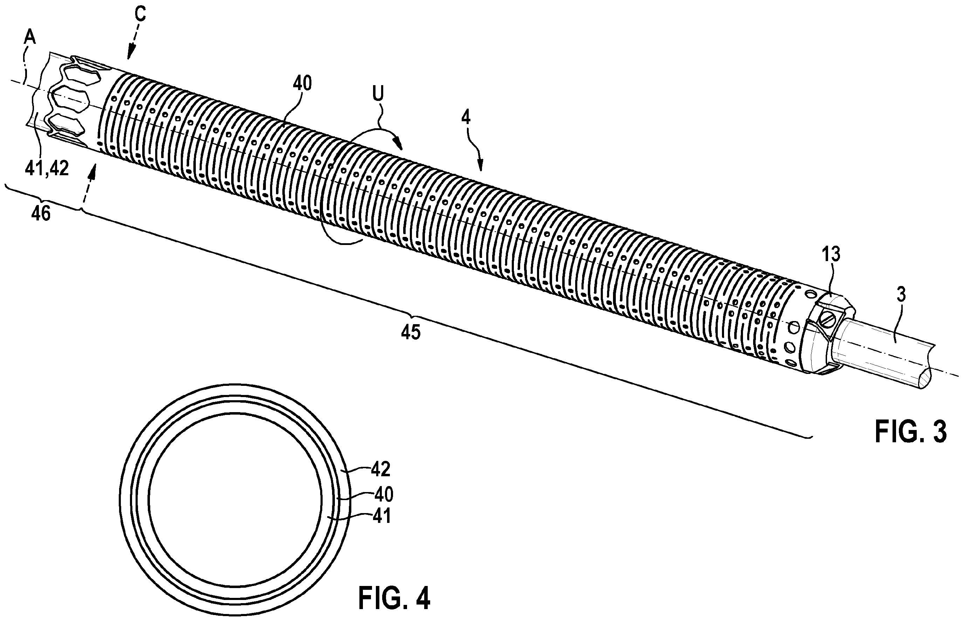

Die Katheterhülse 4 setzt sich zusammen aus einer Versteifungshülse 40, die zwischen einer innenliegenden ersten Polymerlage 41 und einer außen die Versteifungshülse 40 umgebenden zweiten Polymerlage 42 eingebettet ist. Die Polymerlagen 41, 42 ummanteln die Versteifungshülse 40 und stehen an deren distalen Ende über das distale Ende der Versteifungshülse 40 über. Die Versteifungshülse 40 besteht vorzugsweise aus einem metallischen Material (alternativ: steifes Polymermaterial) und weist einen proximalen Abschnitt 45 sowie einen distalen Abschnitt 46 auf, wobei der distale Abschnitt auch als Krone bezeichnet wird. Der proximale Abschnitt 45 ist besonders bevorzugt eine Edelstahlhülse, die teilweise geschlitzt ist. An dem äußersten proximalen Ende des proximalen Abschnitts 45 ist die Versteifungshülse 40 über den proximalen Konnektor 13 mit dem Außenschaft 3 verbunden. Die Mittelachse der Versteifungshülse 40 bildet die Längsrichtung A (siehe Fig. 3) der Versteifungshülse 40 beziehungsweise der Katheterhülse 4.

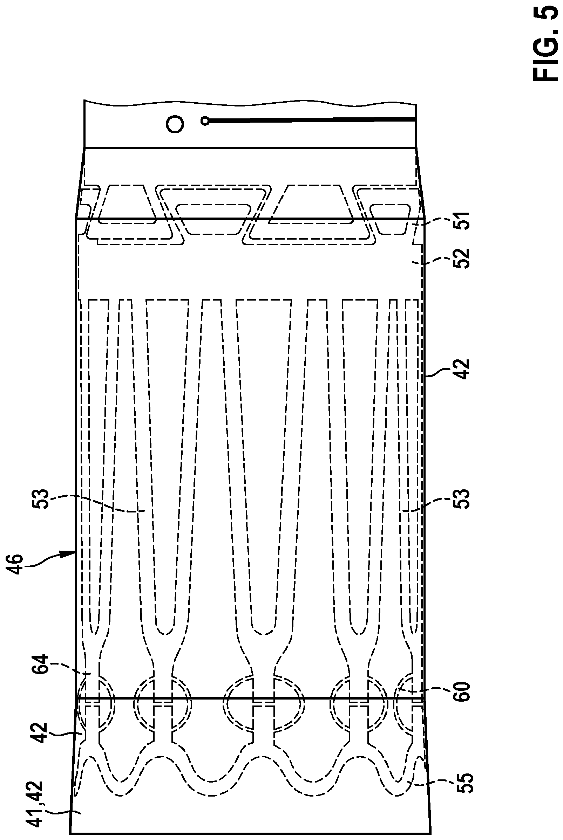

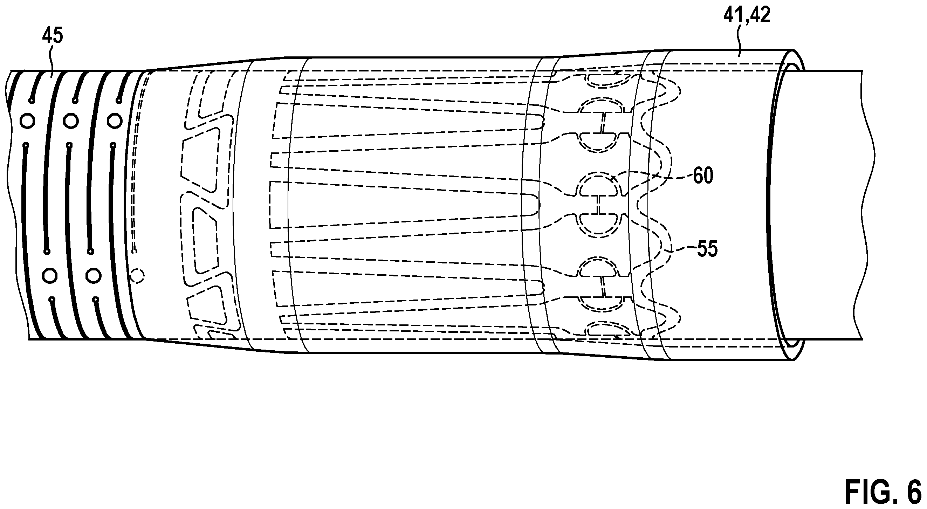

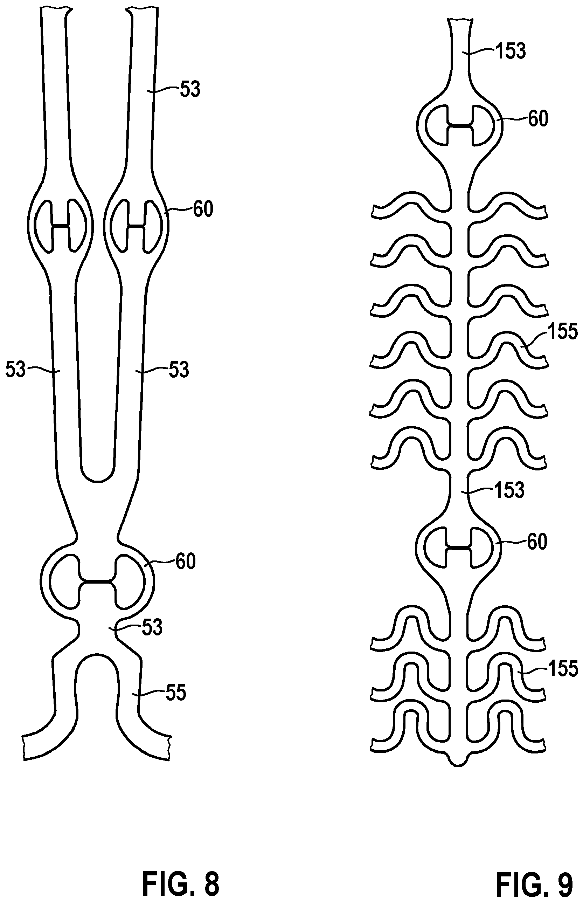

Der distale Abschnitt 46 der Versteifungshülse 40 ist in den Fig. 5 und 6 sowie in einem Ausschnitt in Fig. 8 in einer vergrößerten Abbildung detaillierter gezeigt. Der aus Nitinol bestehende distale Abschnitt 46 weist an seinem proximalen Ende schwalbenschwanzförmige Stege 51 auf, die mit entsprechenden schwalbenschwanzförmigen Einkerbungen des proximalen Abschnitts 45 im Eingriff sind. Im in distaler Richtung sich anschließenden Abschnitt bildet die Struktur einen Ring 52, der in distaler Richtung mit im Wesentlichen in Längsrichtung verlaufenden Stegen 53 verbunden ist. Diese in Längsrichtung verlaufenden Stege 53 sind an ihrem distalen Ende über eine in Umfangsrichtung U verlaufende, über den gesamten Umfang der Versteifungshülse 40 umlaufenden wellenförmigen Steg 55 miteinander verbunden. Die in Längsrichtung verlaufenden Stege 53 sind distal breiter und verlaufen in proximaler Richtung in Form einer zwei-zinkigen Gabel. Im Bereich seiner größeren Breite weist jeder sich in Längsrichtung erstreckender Steg 53 ein Gelenkelement 60 auf, das in den Fig. 8 beziehungsweise 10 noch einmal im Detail dargestellt ist. Fig. 8 zeigt hierbei ein alternatives Ausführungsbeispiel, bei dem auch die schmaleren Gabelstege 53, die in Längsrichtung A verlaufen, jeweils ein Gelenkelement 60 aufweisen.

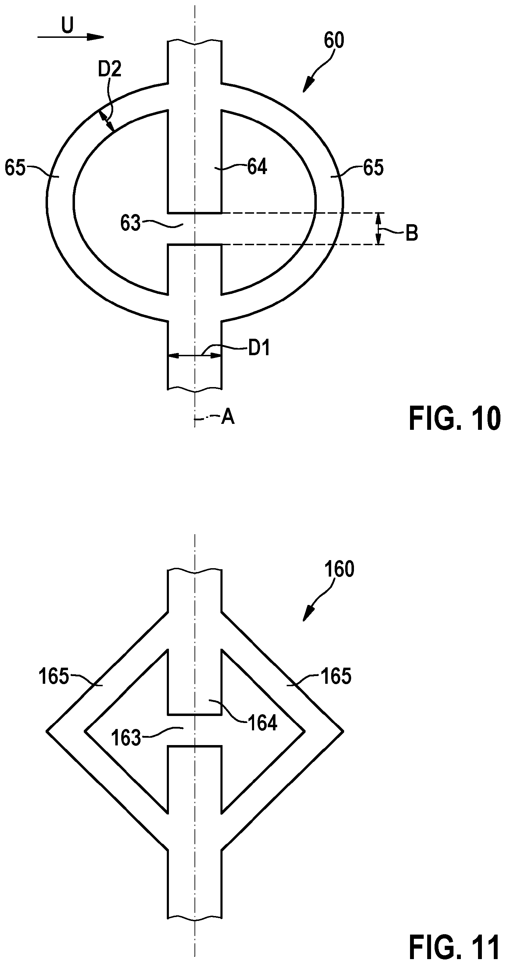

Das in Fig. 10 im Detail gezeigte Gelenkelement 60 weist einen in Längsrichtung A (entspricht der Längsrichtung der röhrenförmigen Struktur der Versteifungshülse 40 beziehungsweise der Katheterhülse 4) verlaufenden Hauptsteg 64 auf. Der Hauptstege 64 bildet in der erfindungsgemäßen Struktur eine Fortsetzung des jeweiligen in Längsrichtung verlaufenden Stegs, der mit dem Bezugszeichen 53 bezeichnet ist. Mittig im Bereich des Gelenkelements 50 ist der Hauptsteg 64 vollständig durchtrennt, so dass ein Spalt 63 entsteht. In Umfangsrichtung U neben dem Spalt 63 beziehungsweise dem Hauptsteg 64 ist auf jeder Seite jeweils ein Brückensteg 65 angeordnet, der jeweils etwa eine Halbkreisform ausbildet und in distaler beziehungsweise proximaler Richtung vor und hinter dem Spalt mit dem Hauptsteg 64 verbunden ist. Das Gelenkelement 60 erlaubt die Übertragung axialer Druckkräfte, d. h. von Druckkräften, die in Längsrichtung A verlaufen, sowie radialer Druckkräfte, so dass der distale Abschnitt 46 eine Aufweitung der Struktur in Form eines Trichters ermöglicht. In Zugrichtung besitzt das Gelenkelemente 60 eine geringere Steifigkeit, der (die) Brückensteg(e) 65 verhindern jedoch ein Auseinanderdriften der Struktur des distalen Abschnitts 46 bei leichten Zugkräften. Abhängig von der Breite B des Spalts 63 von 40 µm - 500 µm kann zusätzlich eine Federfunktion mit Anschlag (Maximalkraft) realisiert werden. Hierdurch weist die erfindungsgemäße Katheterhülse 4 beim Freisetzen beziehungsweise Zurückziehen eines Implantats besonders gute biegeelastische Eigenschaften auf und kehrt vollständig elastisch in seine Ausgangsform zurück, sodass eine Verformung vermieden werden kann.

Bei dem hier dargestellten Ausführungsbeispiel hat der Hauptsteg 64 eine Breite D1 (in Umfangsrichtung U gemessen) von 50 µm - 500 µm und jeder Brückensteg 65 eine Breite D2 von 20% - 50% der Stegbreite des Hauptsteges (senkrecht zur Mittellinie des Brückenstegs 65 gemessen).

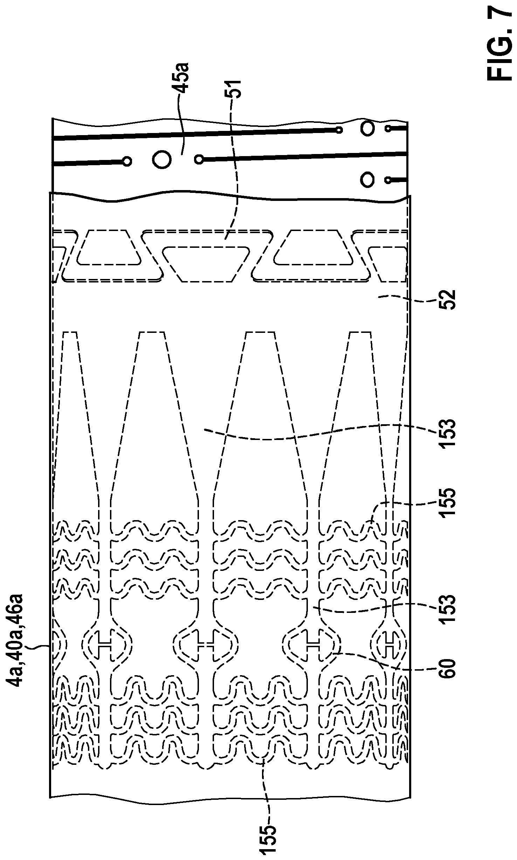

Die Fig. 7 und 9 beziehen sich auf ein zweites Ausführungsbeispiel einer erfindungsgemäßen Katheterhülse 4a mit einer geringfügig geänderten Versteifungshülse 40a. Der distale Abschnitt 46a weist analog zu dem distalen Abschnitt 46 des vorangegangen Ausführungsbeispiels am proximalen Ende schwalbenschwanzförmige Stege 51 zur Verbindung mit dem proximalen Abschnitt 45a der Versteifungshülse 40a auf. In distaler Richtung schließt sich daran ein Ring 52, der durch entsprechende Einkerbungen graduell in Längsrichtung A verlaufende Stege 153 übergeht. Die in Längsrichtung verlaufenden Stege 153 werden mittels einer Vielzahl von in Umfangsrichtung U umlaufenden, wellenförmigen Stegen 155 miteinander verbunden. An dem distalen Ende des distalen Abschnitts 46a zwischen zwei Abschnitten mit wellenförmigen Stegen 155 sind Gelenkelemente 60 vorgesehen, die an jedem in Längsrichtung verlaufenden Steg 153 angeordnet sind und in Umfangsrichtung U nebeneinander liegen. Das Gelenkelement 60 ist in Fig. 10 dargestellt und wurde bereits oben erläutert.

In den Fig. 11 bis 15 sind weitere Ausführungsbeispiele für Gelenkelemente gezeigt. Das in Fig. 11 gezeigte Gelenkelement 160 weist im Unterschied zu dem Gelenkelement 60 der Fig. 10 V-förmige Brückenelemente 155 auf. Bei dem in Fig. 11 dargestellten Ausführungsbeispiel eines Gelenkelements 250 ist der Brückensteg 255 U-förmig gestaltet.

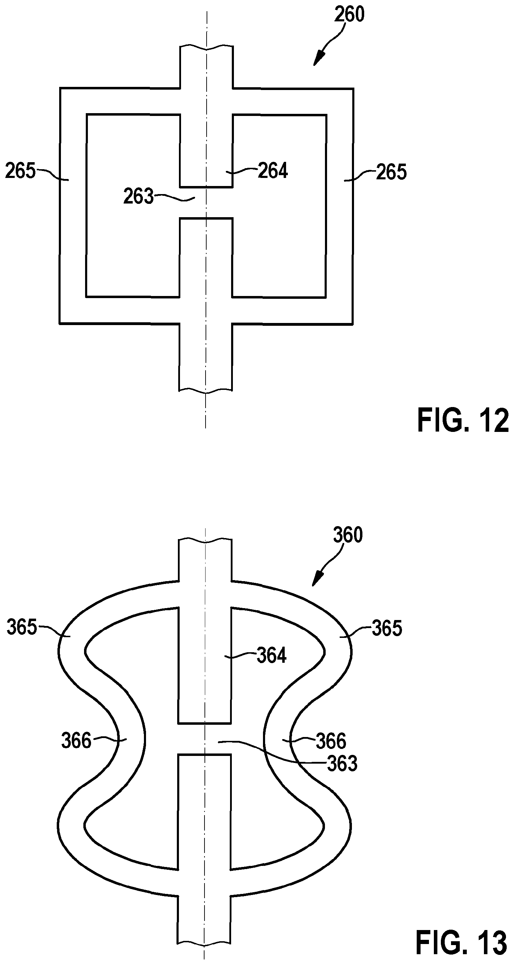

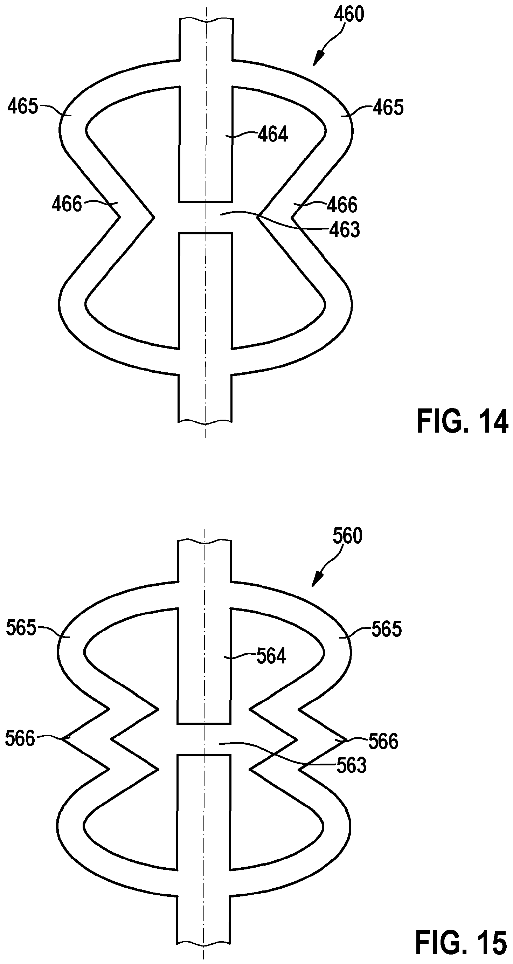

Die in den Fig. 13 bis 15 angegebenen Ausführungsbeispiele für Gelenkelemente 350, 450, 550 ähneln dem in Fig. 10 gezeigten Gelenkelement 50, weisen jedoch etwa auf Höhe des Spalts 355, 455, 555 im Bereich des jeweiligen Brückenstegs 365, 465, 565 jeweils einen Abschnitt auf, der eine Einkerbung zur Erhöhung der Flexibilität besitzt. Dieser Einkerbungsabschnitt kann (nicht dargestellt) lediglich einer Verringerung der Breite oder Dicke des jeweiligen Brückenstegs 365, 465, 565 oder, wie in den Figuren dargestellt, eine Richtungsänderung der Biegung beinhalten. Hierdurch resultiert ein U-förmiger Einkerbungsabschnitt 366 (oder Wellenform, siehe Fig. 13) beziehungsweise ein V-förmige Einkerbungsabschnitt 466 (Fig. 14). Dieser verläuft in Fig. 13 eher abgerundet und in Fig. 14 eher spitz. In Fig. 15 ist der Einkerbungsabschnitt 566 W-förmig gestaltet, was die Federeigenschaft des Brückenstegs 565 erhöht.

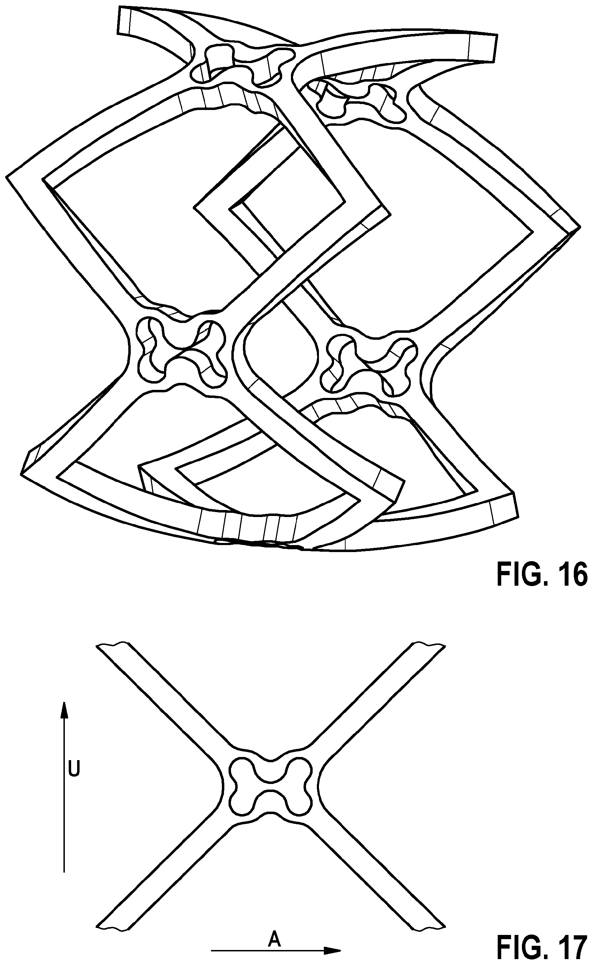

Bei Implantaten wie beispielsweise Stents sind auch Ausführungen denkbar, die Gelenke mit Ausrichtung in Umfangsrichtung U aufweisen derart, dass der Hauptsteg (oder Knoten) in Umfangsrichtung U orientiert ist und hier durch einen Gelenkspalt unterbrochen wird. Eine solche Ausführungsform ist als Stent oder Ausschnitt eines Stents in Fig. 16 gezeigt. Die genaue Ausgestaltung dieses Beispiels für das Gelenk kann Fig. 17 entnommen werden. Es wird ersichtlich, dass die Ausschnittsform des Gelenks einem verkürzten Knochen oder zweier über die Spitzen überlagerte Herzen gleicht. Dabei können die Gelenke auch als Ersatz für Strutkreuzungen verwendet werden, um die Biegeflexibilität in Längs- und Umfangsrichtung zu erhöhen.

Die oben beschriebene erfindungsgemäße Struktur, die in einer Katheterhülse oder in einem Implantat eingesetzt werden kann, ermöglicht die Übertragung großer radialer und axialer Druckkräfte bei gleichzeitig hoher Biegeflexibilität.

### Bezugszeichenliste

- 1: Katheter
- 2a: Handgriff
- 2b: Stabilisierabschnitt
- 3: Außenschaft
- 4, 4a: Katheterhülse
- 7: Innenschaft
- 9: Prothesenkonnektor
- 11: röntgenopaker Ring
- 13: proximaler Konnektor
- 40, 40a: Versteifungshülse
- 41: erste Polymerlage
- 42: zweite Polymerlage
- 45, 45a: proximaler Abschnitt
- 46, 46a: distaler Abschnitt
- 51: schwalbenschwanzförmiger Steg
- 52: Ring
- 53, 153: in Längsrichtung verlaufender Steg
- 55, 155: wellenförmiger Steg
- 60, 160, 260, 360, 460, 560: Gelenkelement
- 63, 163, 263, 363, 463, 563: Spalt
- 64, 164, 264, 364, 464, 564: Hauptsteg
- 65, 165, 265, 365, 465, 565: Brückensteg
- 366, 466, 566: Einkerbungsabschnitt (U-förmig, V-förmig oder W-förmig)
- U: Umfangsrichtung
- A: Längsrichtung der Versteifungshülse 40 oder der Katheterhülse oder eines Implantats
- B: Breite des Spalts
- D1: Breite des Hauptstegs 64
- D2: Breite des Brückenstegs 65

## Patentansprüche

1. Implantat, umfassend eine röhrenförmige durchbrochene Struktur, die sich in Längsrichtung (A) oder in Umfangsrichtung erstreckt und aus einer Vielzahl von Stegen zusammengesetzt ist, die zumindest teilweise in Längsrichtung verlaufen, wobei mindestens ein Gelenkelement (60, 160, 260, 360, 460, 560), dessen im Wesentlichen in Längsrichtung verlaufender Hauptsteg (64, 164, 264, 364, 464, 564) einen durchgehenden Spalt (63, 163, 263, 363, 463, 563) aufweist, wobei das Gelenkelement (60, 160, 260, 360, 460, 560) mindestens einen Brückensteg (65, 165, 265, 365, 465, 565), vorzugsweise zwei Brückenstege, aufweist, wobei jeder Brückensteg (65, 165, 265, 365, 465, 565) in eine Umfangsrichtung (U) der röhrenförmigen Struktur neben dem Hauptsteg (64, 164, 264, 364, 464, 564) angeordnet sowie in Längsrichtung (A) vor und hinter dem Spalt (63, 163, 263, 363, 463, 563) mit dem Hauptsteg (64, 164, 264, 364, 464, 564) verbunden ist,
**dadurch gekennzeichnet, dass**
die Breite (D2) des Brückenstegs (65, 165, 265, 365, 465, 565) mindestens 20% der Stegbreite des Hauptsteges entspricht, und die Summe der Breite aller Brückenstege eines Gelenkelements die Breite des Hauptsteges nicht überschreitet.

2. Implantat nach Anspruch 1, **dadurch gekennzeichnet, dass** der Brückensteg (65, 165, 265, 365, 465, 565) halbkreisförmig, U-förmig, V-förmig oder mäanderförmig ausgebildet ist.

3. Implantat nach Anspruch 1 oder 2, **dadurch gekennzeichnet, dass** der Brückensteg (65, 165, 265, 365, 465, 565) mindestens einen Einkerbungsabschnitt (366, 466, 566) aufweist.

4. Implantat nach einem der vorhergehenden Ansprüche, **dadurch gekennzeichnet, dass** der Spalt (63, 163, 263, 363, 463, 563) eine Breite (B) von mindestens 20 µm, vorzugsweise von mindestens 40 µm - 500 µm, aufweist.

5. Implantat nach einem der vorhergehenden Ansprüche, **dadurch gekennzeichnet, dass** eine Vielzahl von Gelenkelementen (60, 160, 260, 360, 460, 560) in Umfangsrichtung (U) nebeneinander angeordnet sind.

6. Implantat nach einem der vorhergehenden Ansprüche, **dadurch gekennzeichnet, dass** diese aus einer Formgedächtnislegierung, bevorzugt Nitinol, besteht.

7. Implantat nach einem der vorhergehenden Ansprüche, wobei das Implantat ein Stent ist, und **weiter dadurch charakterisiert, dass** der Stent zumindest in einem Abschnitt eine röhrenförmige durchbrochene Struktur gemäß einem der vorhergehenden Ansprüche aufweist.

8. Implantat nach einem der vorhergehenden Ansprüche, **dadurch gekennzeichnet, dass** der mindestens eine Hauptsteg in Längsrichtung verläuft.

9. Implantat nach einem der vorhergehenden Ansprüche, **dadurch gekennzeichnet, dass** mehrere Hauptstege parallel zur Längsrichtung der Struktur verlaufen.

10. Implantat nach einem der vorhergehenden Ansprüche, **dadurch gekennzeichnet, dass** das Gelenkelement eine Struktur ist, welche Druckkräfte übertragen kann, jedoch auch ein Gleiten im Bereich des im Hauptsteg angeordneten Spalts sowie eine Verdrehung der im Spalt gegenüber liegenden Enden des Hauptstegs zueinander erlaubt.

11. Implantat nach einem der vorhergehenden Ansprüche, **dadurch gekennzeichnet, dass** das Gelenkelement ferner in der Lage ist, Druckkräfte, die in Längsrichtung oder in radialer Richtung verlaufen, zu übertragen.

12. Implantat nach einem der Ansprüche 1 bis 2 oder 4 bis 11, **dadurch gekennzeichnet, dass** der Brückensteg mindestens einen Einkerbungsabschnitt mit einer quer zur Längsrichtung verlaufende Einkerbung aufweist.

13. Implantat nach einem der Ansprüche 3 oder 12, **dadurch gekennzeichnet, dass** der Einkerbungsabschnitt als U-, V- oder W-förmiger Abschnitt ausgebildet ist.

14. Implantat nach einem der vorhergehenden Ansprüche, **dadurch gekennzeichnet, dass** in Längsrichtung eine Vielzahl von Gelenkelementen hintereinander angeordnet sind.

## Claims

1. Implant comprising a tubular perforated structure extending in the longitudinal direction (A) or in the circumferential direction and composed of a plurality of bars which extend at least partially in the longitudinal direction, wherein at least one joint element (60, 160, 260, 360, 460, 560), the main web of which (64, 164, 264, 364, 464, 564) extending substantially in the longitudinal direction has a continuous gap (63, 163, 263, 363, 463, 563), wherein the joint element (60, 160, 260, 360, 460, 560) has at least one bridge web (65, 165, 265, 365, 465, 565), preferably two bridge webs, wherein each bridge web (65, 165, 265, 365, 465, 565) is arranged in a circumferential direction (U) of the tubular structure next to the main web (64, 164, 264, 364, 464, 564) and is connected to the main web (64, 164, 264, 364, 464, 564) in the longitudinal direction (A) in front of and behind the gap (63, 163, 263, 363, 463, 563),
**characterized in that**
the width (D2) of the bridge web (65, 165, 265, 365, 465, 565) corresponds to at least 20% of the web width of the main web, and the sum of the width of all bridge webs of an articulated element does not exceed the width of the main web.

2. Implant according to claim 1, **characterized in that** the bridge bar (65, 165, 265, 365, 465, 565) is semicircular, U-shaped, V-shaped or meander-shaped.

3. Implant according to claim 1 or 2, **characterized in that** the bridge bar (65, 165, 265, 365, 465, 565) has at least one notch section (366, 466, 566).

4. Implant according to one of the preceding claims, **characterized in that** the gap (63, 163, 263, 363, 463, 563) has a width (B) of at least 20 µm, preferably of at least 40 µm - 500 µm.

5. Implant according to one of the preceding claims, **characterized in that** a plurality of joint elements (60, 160, 260, 360, 460, 560) are arranged next to one another in the circumferential direction (U).

6. Implant according to one of the preceding claims, **characterized in that** it consists of a shape memory alloy, preferably nitinol.

7. Implant according to any one of the preceding claims, wherein the implant is a stent, and **further characterized in that** the stent comprises at least in one portion a tubular perforated structure according to any one of the preceding claims.

8. Implant according to one of the preceding claims, **characterized in that** the at least one main bar extends in the longitudinal direction.

9. Implant according to one of the preceding claims, **characterized in that** several main bars run parallel to the longitudinal direction of the structure.

10. Implant according to one of the preceding claims, **characterized in that** the joint element is a structure which can transmit compressive forces, but also permits sliding in the region of the gap arranged in the main bar and rotation of the ends of the main bar opposite each other in the gap.

11. Implant according to one of the preceding claims, **characterized in that** the articulation element is further capable of transmitting compressive forces extending in the longitudinal direction or in the radial direction.

12. Implant according to one of claims 1 to 2 or 4 to 11, **characterized in that** the bridge bar has at least one indentation section with an indentation running transversely to the longitudinal direction.

13. Implant according to one of claims 3 or 12, **characterized in that** the notch section is designed as a U-, V- or W-shaped section.

14. Implant according to one of the preceding claims, **characterized in that** a large number of joint elements are arranged one behind the other in the longitudinal direction.

## Revendications

1. Implant comprenant une structure tubulaire ajourée s'étendant longitudinalement (A) ou circonférentiellement et composée d'une pluralité d'entretoises s'étendant au moins partiellement longitudinalement, dans lequel au moins un élément d'articulation (60, 160, 260, 360, 460, 560), dont dans l'âme principale s'étend sensiblement dans la direction longitudinale (64, 164, 264, 364, 464, 564) présente une fente traversante (63, 163, 263, 363, 463, 563), dans lequel l'élément d'articulation (60, 160, 260, 360, 460, 560) présente au moins une barre de pontage (65, 165, 265, 365, 465, 565), de préférence deux barres de pontage, dans lequel chaque barre de pontage (65, 165, 265, 365, 465, 565) est disposée à côté de l'âme principale (64, 164, 264, 364, 464, 564) dans une direction circonférentielle (U) de la structure tubulaire et est reliée à l'âme principale (64, 164, 264, 364, 464, 564) dans la direction longitudinale (A) en avant et en arrière de l'espace (63, 163, 263, 363, 463, 563),
**caractérisé en ce que**
la largeur (D2) de l'âme de pont (65, 165, 265, 365, 465, 565) correspond à au moins 20% de la largeur de l'âme de l'âme principale, et la somme de la largeur de toutes les âmes de pont d'un élément d'articulation ne dépasse pas la largeur de l'âme principale.

2. Implant selon la revendication 1, **caractérisé en ce que** la barre de pontage (65, 165, 265, 365, 465, 565) a une forme semi-circulaire, en U, en V ou en méandres.

3. Implant selon la revendication 1 ou 2, **caractérisé en ce que** la barre de pontage (65, 165, 265, 365, 465, 565) présente au moins une section d'encoche (366, 466, 566).

4. Implant selon l'une des revendications précédentes, **caractérisé en ce que** l'espace (63, 163, 263, 363, 463, 563) présente une largeur (B) d'au moins 20 µm, de préférence d'au moins 40 µm - 500 µm.

5. Implant selon l'une des revendications précédentes, **caractérisé en ce qu'**une pluralité d'éléments d'articulation (60, 160, 260, 360, 460, 560) sont disposés côte à côte dans la direction circonférentielle (U).

6. Implant selon l'une des revendications précédentes, **caractérisé en ce qu'**il est constitué d'un alliage à mémoire de forme, de préférence du nitinol.

7. Implant selon l'une quelconque des revendications précédentes, dans lequel l'implant est un stent, et **caractérisé en outre en ce que** le stent présente, au moins dans une partie, une structure tubulaire ajourée selon l'une quelconque des revendications précédentes.

8. Implant selon l'une des revendications précédentes, **caractérisé en ce que** ladite au moins une barre principale s'étend dans la direction longitudinale.

9. Implant selon l'une des revendications précédentes, **caractérisé en ce que** plusieurs barrettes principales sont parallèles à la direction longitudinale de la structure.

10. Implant selon l'une des revendications précédentes, **caractérisé en ce que** l'élément d'articulation est une structure qui peut transmettre des forces de compression, mais qui permet également un glissement dans la zone de la fente disposée dans la barre principale ainsi qu'une rotation des extrémités de la barre principale opposées l'une à l'autre dans la fente.

11. Implant selon l'une quelconque des revendications précédentes, **caractérisé en ce que** l'élément d'articulation est en outre capable de transmettre des forces de compression qui s'étendent dans la direction longitudinale ou dans la direction radiale.

12. Implant selon l'une des revendications 1 à 2 ou 4 à 11, **caractérisé en ce que** la barre de pontage présente au moins une portion d'encoche avec une encoche s'étendant transversalement à la direction longitudinale.

13. Implant selon l'une des revendications 3 ou 12, **caractérisé en ce que** la section d'encoche est une section en forme de U, de V ou de W.

14. Implant selon l'une des revendications précédentes, **caractérisé en ce que** une pluralité d'éléments d'articulation sont disposés les uns derrière les autres dans le sens de la longueur.
